# EUROPEAN PATENT SPECIFICATION

(11) **EP 3 068 284 B1**
(45) Date of publication and mention of the grant of the patent: **02.01.2019**
(21) Application number: 14727921.0
(22) Date of filing: 18.04.2014
(51) Int. Cl.: A61B 3/12, A61B 3/14, G03B 17/56

(54) **OPTICAL ACCESSORY FOR A MOBILE DEVICE**
OPTISCHES ZUBEHÖR FÜR EINE MOBILE VORRICHTUNG
ACCESSOIRE OPTIQUE POUR DISPOSITIF MOBILE

(30) Priority: 15.11.2013 IT BS20130169
(43) Date of publication of application: 21.09.2016
(73) Proprietor: D-EYE S.r.l., 35131 Padova (IT)
(72) Inventor: RUSSO, Andrea, 25128 Brescia (BS) (IT)
(74) Representative: Pes, Matteo
(86) International application number: PCT/IB2014/060842
(87) International publication number: WO 2015/071779

(56) References cited:
- WO-A1-2012/039998
- JP-A- 2004 279 733
- US-A1- 2011 109 971
- US-A1- 2013 083 185

## Description

### Field of the invention

The present invention is part of the field of optical instruments used in particular, but not exclusively, in the medical industry and specifically relates to an optical accessory applied to a mobile device provided with a camera, in particular a smartphone or tablet, to realize an ophthalmoscope.

### State of the art

As known, the ophthalmoscope is an optical device allowing a doctor to view the eyeground or "*fundus oculi*" of a human eye, in order to diagnose ocular diseases (glaucoma, macular degeneration, venous and arterial occlusions, etc.) and/or systemic diseases (diabetes, arterial hypertension, intracranial hypertension, etc.).

Nowadays, this examination is carried out in eye care clinics by means of non-portable ophthalmoscopic instruments positioned on bulky and expensive supporting structures which implement both optical and computer equipments assisting the optical equipment. However, this type of instruments has proved to be impractical and greatly limiting the observation of the "*fundus oculi*", in particular in bedridden, paediatric, disabled patients, or in any case patients who cannot immediately access an eye care clinic.

To overcome the aforesaid drawbacks, portable ophthalmoscopes have been developed, as described for example in US2013128223.

These instruments mainly comprise a lighting system having at least a light source for illuminating the eye of a patient. A binocular vision system used by the doctor and associated to the lighting system allows to view the eye as illuminated by the light source.

With the recent coming of smartphones and tablets provided with cameras, the portable ophthalmoscope was associated to these mobile devices by positioning a detachable support. In particular, the support is adapted to house the mobile device such that the camera can be positioned at the observing portion of the ophthalmoscope, i.e. the portion intended for allowing the user to carry out the observation. In this way, the camera acts as a digital-image acquisition device; however, the light source is still provided by the instrument.

Such a system can be seen, for example, in the website *www*.*welchallyn.com*/*promotions*/*iExaminer*/*index.html.*

Diffusion and the usability of this system are limited because it is very expensive, being further bulky and not very ergonomic.

In addition, the application of the mobile device only as an image acquisition device functionally restricts its use, in particular as regards the extension of the visual field that can be obtained and, therefore, the accuracy of the examination.

In addition, for unskilled personnel is very difficult to use this system. In particular, the support of the mobile device can be misaligned with respect to the instrument, thereby requiring frequent adjustments of the position.

JP 2004 279733 A discloses a unit for magnification imaging mounted on a portable telephone with a camera having a first lens, an image pickup element and an illumination, in which the unit for magnification imaging is configured by housing a magnification lens, a first reflecting mirror and a second reflecting mirror inside a main body whose front face is formed with an abutting part and whose back face is formed with an opening and a second member by which the magnification imaging unit is detachably mounted on the portable telephone, in such a way that when the unit for magnification imaging is mounted on the portable telephone, the magnification lens is positioned on the front face of the first lens so that a magnified image can be imaged, and in which an illuminating light from the illumination is successively reflected on the second reflecting mirror and the first reflecting mirror so as to be introduced to an object to be imaged so that it is possible to illuminate the object to be imaged.

US 2013/083185 A1 discloses an optical adapter system for a smart-phone, having a lens and a light transmission guide within a housing, in which the lens is located at the distal end of the housing in optical alignment with the smart-phone's camera lens, and the light transmission guide extends from one end that is adjacent to the smart-phone's light source toward the distal end of the housing and directs light outside of the housing to the subject being imaged, in which the optical adapter is used in combination with the smart-phone as an ophthalmic examination tool and can be used with a computer program that runs on the smart-phone's processor.

WO 2012/039998 A1 discloses an adaptor that adapts an SLR camera for use as an ophthalmic viewing and imaging device, in which the adaptor is mounted to the camera between the camera body and the camera lens, in which the adaptor includes a first light source and in which the adaptor directs light from a second, external source, in which optical components within the adaptor define a first optical pathway for directing light from the first light source to the lens, a second optical pathway for directing light from the second light source to the camera lens, and a third optical pathway for directing light from the camera lens through the adaptor to the camera.

US 2011/109971 A1 discloses systems that divert images for image capture devices, in which an image diversion system includes a deviating optical element to provide a first view to an imager of an image capture device and to provide at least a second view to the imager, or in which an image diversion system includes a first deviating optical element and a second deviating optical element optically coupled to the first deviating optical element in which the first deviating optical element provides a first view and at least a second view to an imager of an image capture device, or in which the image diversion system includes a first deviating optical element, a second deviating optical element, and at least one corrective lens, and in which the image diversion system is contained within a housing that is attachable to a housing portion of an image capture device.

### Summary of the invention

It is therefore an object of the present invention, which is defined in independent claim 1 and the dependent claims 2 and 3, to provide an optical accessory for a mobile device provided with a camera which is able to solve the problems of the known art systems.

It is another object of the present invention to provide an optical accessory smaller than known devices.

It is a further object of the present invention to provide an optical accessory for a mobile device of simple structure and being cost effective.

These and other objects are achieved by an optical accessory for a mobile device according to claim 1. Mobile device means a smartphone, a tablet or another similar electronic device provided with a camera, as described below.

In particular, the optical accessory comprises coupling means to be functionally attached to the mobile device thereby substantially forming an ophthalmoscope, as described below.

By the coupling means the optical accessory can be removably attached in correspondence with a camera lens and a respective light source provided by the mobile device. Camera lens means a lens able to capture digital images in form of photographs or videos.

An optical accessory which is not covered by the present invention comprises a first optical element provided with a first reflective surface, in particular a mirror, apt to reflect a source light beam emitted by the light source and to generate a first reflected beam directed toward the visual field of the camera lens. In other words, the mirror only reflects, or deviates, the source light beam in the visual field of the camera lens.

In addition, the accessory comprises a second optical element, in particular a beam splitter, comprising a partially reflective second surface, preferably parallel to the first reflective surface. The second surface, on which the first reflected beam is incident, generates a second reflected beam directed outside the accessory toward an eye to be examined. The second reflected beam is substantially inside the visual field of the camera lens, i.e. substantially aligned with it to allow the view of the "*fundus oculi*" of the eye itself.

In particular, by positioning the optical accessory applied to the mobile device close to the eye to be examined so that the second light beam is incident on the eye, the eye can be viewed and its digital images can be captured, through the camera lens.

In particular, the first surface is a flat surface and is inclined at an angle α comprised between 30° and 60° with respect to a lying plane P of the said camera lens and said light source. Also the second surface is a flat surface inclined at an angle α' comprised between 30° and 60° with respect to the lying plane P.

Preferably, the first surface is inclined at about 45° with respect to the plane P and the second surface is parallel to the first surface and also inclined at 45°. In this way, the source light beam emitted by the light source, generally a LED source, is reflected by 90° toward the visual field in front of the camera lens.

Preferably, the first surface is inclined at about 52.5° with respect to the plane P and the second surface is parallel to the first surface and also inclined at 52.5°.

In this way, the source light beam emitted by the light source, generally a LED source, is reflected by 97.5° toward the visual field in front of the camera lens. In front of the camera lens the *beam splitter* is positioned, having the partially reflective surface parallel to the first surface of the mirror. The first reflected beam is incident on the second surface of the *beam splitter* and generates, respectively, a transmitted beam passing through the beam splitter and a second reflected beam directed outside of the accessory for illuminating an eye to be examined.

The second reflected beam is substantially inside the visual field of the camera lens and, once positioned in-line with the eye, allows to carry out visual examination of the "*fundus oculi*" and its image to be captured by the lens.

Since the above mentioned system uses both the camera lens and the light source of the mobile device, i.e. of the smartphone, it is a small accessory easy to be used even by unskilled personnel.

Preferably, a light-absorbing wall is provided which is associated to the second optical element and placed opposite to the first optical element. The light-absorbing wall substantially allows to absorb the transmitted beam passing through the beam splitter in order to reduce the phenomena of reflection and loss of contrast.

Advantageously, the light-absorbing wall is inclined at 45° and is specularly opposed with respect to the inclination of the first and second optical elements.

Preferably, a diaphragm having a hole is comprised between the first optical element and the second optical element. The diaphragm allows to collimate the first beam reflected by the mirror and directed toward the beam splitter.

In a preferred embodiment, the coupling means have the shape of a cover attachable to the mobile device integrating said optical accessory.

Preferably, a first polarizing filter disposed between the diaphragm and the second optical element, and a second polarizing filter disposed in front of the camera lens, are provided. The polarizing filters allow to reduce the light reflex on the cornea of the eye, most of all when the patient has miotic pupils.

In an embodiment according to the present invention, said first and second optical elements have the shape of an optical guide element comprising at least one first face identifying said first reflective surface, and at least one second face identifying said partially reflective second surface.

In particular, said optical guide element is a monoblock made of a transparent material, wherein said first face has a polished surface and said at least one second face has a satin-finished surface which allows to generate said second light beam.

In detail, in this embodiment according to the present invention, the source light beam strikes the first face of the optical guide element thereby generating the first reflected ray. The first reflected ray propagates into the optical guide element being subjected - in turn - to a plurality of total reflections before impinging on the second face of the optical guide element and generating the second reflected beam. The structure itself of the optical guide element allows to have total reflections of the first reflected ray. Therefore, in this arrangement, the term "first reflected ray" means the ray generated by the reflection with the first face, which is subjected to further inner reflections propagating into the optical element, until it impinges on the partially reflective second face of the same.

In particular, said first face is inclined at an angle φ comprised between 30° and 60° with respect to the lying plane (P).

In particular, said second face is inclined at an angle γ comprised between 30° and 60° with respect to the lying plane (P).

Preferably, the first and second faces of the optical guide element are inclined oppositely relative to one another. The optical guide element has a substantially trapezoidal longitudinal section.

In a further embodiment according to the present invention, said first optical element is an optical guide element comprising at least one first face identifying said first reflective surface and a second face opposite to the first face. The second face has a transparent surface. On the contrary, the second optical element is a beam splitter positioned in correspondence with the second face of said optical guide element, such that the generated second reflected beam is directed coaxially with the axis of the eye to be observed.

In this modification, the first and second faces of the second optical guide element are inclined in the same direction, in particular the first and second faces are parallel to each other.

More particularly, the first reflected beam outgoing from the optical guide element, and specifically the second face, is substantially directed in the opposite direction with respect to the eye to be observed. The first reflected beam impinges on the beam splitter for generating the second reflected beam directed toward the eye to be observed. By positioning the beam splitter substantially in front of the lens, a second reflected beam in-line with the eye and the lens can be generated. This leads to the advantage of reducing the corneal reflex of the eye itself on the lens.

In a described embodiment which is not covered by the present invention, said first optical element is an optical fibre and said second optical element is a reflective element, in particular a prism or a mirror.

The reflective element, on which the first light beam outgoing from the optical fibre is incident, generates the second light beam directed in the optical field of the lens, illuminating the eye to be observed.

Preferably, at the exit of the optical fibre, a first polarizing filter for said first light beam is provided.

The second generated light beam, which strikes the eye to be observed, is inclined with respect to the optical axis. Advantageously, in the visual field of the lens, a second polarizing filter is provided, allowing to reduce the corneal reflex of the eye itself.

In a described embodiment, which is not covered by the present invention, said first optical element is an optical fibre and said second optical element comprise said reflective element in turn associated to a beam splitter, such that the second light beam is reflected coaxially with the axis of the eye to be observed.

Moreover, in this way, only the normal polarization reaches the user eye, so that the corneal reflex of the eye itself is reduced.

Preferably, also in this modification, the first polarizing filter is provided at the exit of the optical fibre.

According to another aspect of the invention an optical device comprising an optical accessory according to claim 1 or 2, functionally associated to a mobile device, such as a smartphone or tablet having a camera lens and a light source, is provided.

### Brief list of the figures

More features and advantages of the invention will be better understood by considering the following specification of several, but not exclusive, preferred embodiments, illustrated by way of example only and without limitation, with the support of the accompanying drawings, in which:
- figure 1 is a perspective view of an optical accessory which is not covered by the present invention, applied to a mobile device, as a smartphone or tablet;
- figure 2 is a schematic view of the optical accessory of figure 1;
- figure 3 is a perspective view of the optical accessory of figure 1;
- figure 4 shows a schematic view of an embodiment of an optical accessory according to the present invention;
- figure 5 shows a further embodiment of the optical accessory according to the invention;
- figure 6 shows a schematic view of an embodiment which is not covered by the present invention;
- figure 7 shows a schematic view of an embodiment which is not covered by the present invention.

### Detailed description of the invention

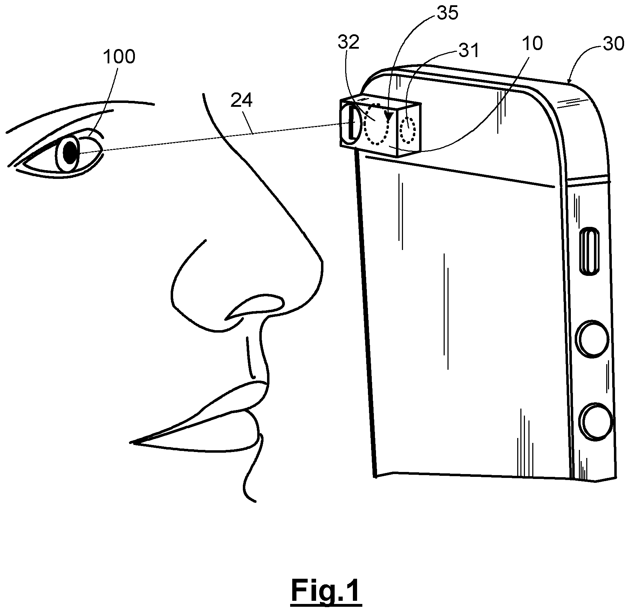

Referring to figure 1, an optical accessory 10 applied to a mobile device 30, as a smartphone or tablet, is shown. The mobile device 30 comprises a camera 35 provided with a camera lens 32 and a light source 31, for example a LED source.

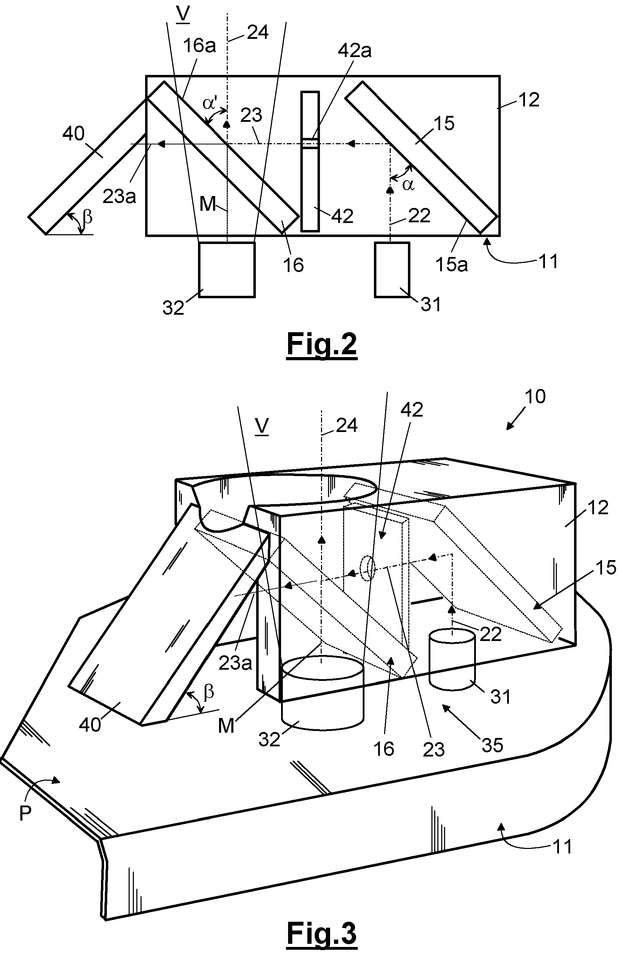

As better schematically shown in figures 2 and 3, the optical accessory 10 is provided with coupling means 11 shaped so as to connect and position it in correspondence with the lens 32 and the light source 31 of the mobile device 30. For example, the coupling means 11 can be formed by a simple adhesive surface or a cover 11 (fig. 3) to be snap coupled to the mobile device 30.

More particularly, the optical accessory 10 comprises a substantially box-shaped frame 12 in which a first optical element 15 provided with a first reflective surface 15a, in particular a mirror, is positioned. The surface 15a of the mirror 15 allows to reflect a source light beam 22 emitted by the light source 31. The total reflection of the source beam 22 generates a first reflected beam 23 directed toward the visual field V of the camera lens 32. In other words, the mirror 15 reflects the light beam in an area in front of the lens 32.

The accessory 10 further comprises a second optical element, in particular a *beam splitter* 16 provided with a partially - reflective second surface 16a. The first reflected beam 23 is incident on the surface 16a of the *beam splitter* 16 and it generates a second reflected beam 24 and a transmitted beam 23a.

The second surface 16a is oriented such as to be substantially parallel to the first surface 15a of the mirror 15. Both the surface 15a of the mirror 15 and the surface 16a of the *beam splitter* 16 are preferably flat surfaces.

The so-generated second beam 24 outgoing from the *beam splitter* 16 is directed to illuminate the patient eye 100 (fig. 1) to be observed and is substantially inside the visual field V of the camera lens 32 and, in particular, is aligned to an axis or centerline M of the latter.

By positioning the optical accessory 10, associated to the mobile device 30, close to the eye 100 so that the light beam 24 is substantially on its optical axis, it is possible to carry out the examination of the eyeground or "*fundus oculi*" of the eye 100; at the same time, the optical accessory is simple, functional, portable and compact. With the optical accessory 10 assembled to the mobile device 30, in fact, the examination can be carried out very simply even through miotic pupils.

According to structural details, still referring to figures 2 and 3, the surface 15a of the mirror 15 is inclined at an angle α of about 45°, in particular of about 52.5°, with respect to the lying plane P (fig. 3) of the lens 32 and the light source 31, or, with respect to the source light beam 22, as shown in figure 2. The lying plane P is substantially the outer flat surface of the mobile device 30. The *beam splitter* 16 parallel to the mirror 15 is also inclined at an angle α' of about 45°, in particular of 52.5°. In this way, the source light beam 22 is reflected by 90°, in particular by 97.5°, thereby generating the first reflected beam 23. Similarly the first beam 23, when it strikes the surface 16a of the *beam splitter* 16, is reflected by 90° and generates the second reflected beam 24 which strikes the eye 100.

The range of inclination of the α, α' angles relative to the surfaces of the mirror 15 and the beam splitter 16 can change and being comprised preferably between 30° and 60°.

Advantageously, as above said, once the optical accessory 10 is positioned in line with the eye 100, the beam splitter 16 in front of the camera lens 32, i.e. in line with the centerline M of the visual field V, allows to carry out the examination of the eyeground and capture an image thereof by the lens 32.

In a preferred embodiment, the optical accessory 10 further comprises a light-absorbing wall 40 inclined at an angle β comprised between 30° and 60°, preferably of 45°. The inclination of the wall 40 is specularly opposed with respect to the inclination of the *beam splitter* 16 and the mirror 15. The light-absorbing wall 40 is placed opposite with respect to the mirror 15 and substantially allows to absorb the transmitted beam 23a passing through the *beam splitter* 16 and reduce the phenomena of reflection and loss of contrast of the lens 32.

Further, the accessory provides a diaphragm 42 provided with a calibrated hole 42a interposed between the mirror 15 and the *beam splitter* 16 on the path of the first reflected beam 23. The diaphragm 42 allows to substantially collimate the first reflected beam 23 and reduce its dispersion.

Still advantageously, in a way not shown in detail, the optical accessory 10 comprises a first polarizing filter disposed between the diaphragm 42 and the *beam splitter* 16 and a second polarizing filter disposed in front of the camera lens 32. These are advantageously employed to reduce the phenomena of light reflection on the cornea of the eye 100, especially in patients with miotic pupils.

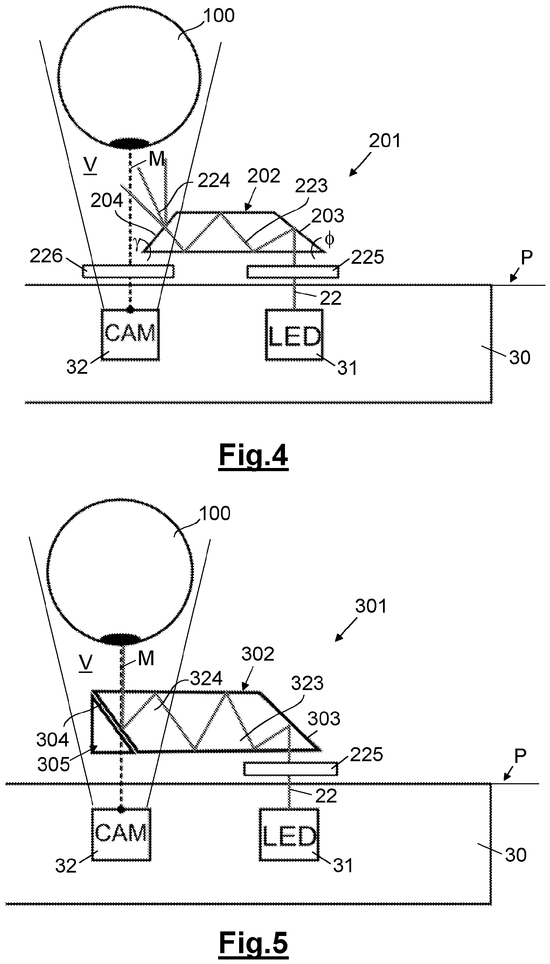

Referring to figure 4, an embodiment according to the present invention of the optical accessory is shown, generally referred to with the numeral 201, wherein parts structurally and functionally corresponding to the optical accessory 10, which is illustrated with reference to figures 1 to 3, maintain the already used numerals.

In this embodiment according to the invention, the first and the second optical elements have the shape of an optical guide element 202 comprising at least one first face 203 identifying a first reflective surface, and at least one second face 204 identifying a partially reflective second surface.

In particular, the optical guide element 202 is a monoblock made of a transparent material, in which the first face 203 has a polished surface whereas the second face 204 has a satin-finished surface, that allows to generate a second substantially-divergent light beam 224 directed toward the eye 100 to be observed, always inside the visual field V of the lens.

In more details, the optical guide element is made of a transparent and homogeneous material, for example plastic (PMMA, polycarbonate, etc.) or glass.

According to the operation principle, the source light beam 22 strikes the first face 203 of the optical guide element 202 thereby generating the first reflected ray 223. The first reflected ray 223 propagates in the optical guide element 202 being subjected - in turn - to a plurality of total reflections before impinging on the second face 204 of the partially-reflective optical guide element and generating the second reflected beam 224. The structure itself of the optical guide element 202 allows to have total reflections of the first reflected ray 223.

Structurally, the first face 203 is inclined at an angle φ comprised between 30° and 60° with respect to the lying plane P, while the second face 204, being oriented oppositely with respect to the first face 203, preferably is inclined at an angle γ comprised between 30° and 60° with respect to the lying plane P.

In a further embodiment of the accessory according to the present invention, generally referred to as 301 and shown in figure 5, the first optical element is an optical guide element 302 structurally shaped as the optical guide element 202 of figure 4, which comprises at least one first face 303 identifying the first reflective surface and a substantially transparent second face 304. On the contrary, the second optical element is a beam splitter 305 positioned in correspondence with the second face 304. By positioning the beam splitter 305, a second reflected beam 324 can be generated and directed coaxially with the axis of the eye M to be observed.

Structurally, the first face 303 and second face 304 of the optical guide element 302 are concordantly inclined on the same side and, in particular, are substantially parallel to each other. The first reflected beam 323 outgoing from the optical guide element 302 is directed substantially in the opposite direction with respect to the eye 100 to be observed. This beam reflects on the beam splitter 305 thereby generating the second reflected beam 324 directed toward the eye 100. The second reflected beam 324 is coaxial with the axis M of the eye and with the lens 32 in the visual field V, thereby leading to the advantage of reducing the corneal reflex of the eye itself on the lens.

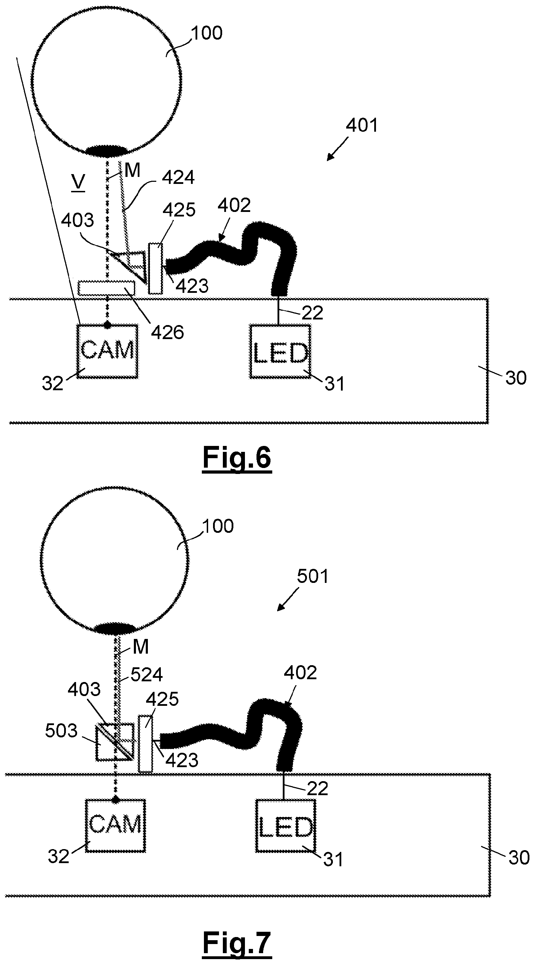

In an embodiment with reference to figure 6, which is not covered by the present invention, an optical accessory generally referred to as 401 is shown, providing an optical fibre 402 which is the first optical element, and a reflective element 403, particularly a prism, which is the second optical element. Reflective element means an at least partially-reflective element used to orient and split the light beam.

The reflective element 403, on which the first light beam 423 outgoing from the optical fibre 402 is incident, generates the second light beam 424 directed in the optical field V of the lens 32, which illuminates the eye 100 to be observed.

The second light beam 424 is inclined with respect to the optical axis M.

In this modification, at the exit of the optical fibre 402 a first polarizing filter 425 of the first light beam 423 is provided, as well as a second polarizing filter 426 positioned in front of the lens 32 for reducing the phenomena of corneal reflection of the eye 100 itself.

In an embodiment shown in figure 7 which is not covered by the present invention, the optical accessory, generally referred to as 501, comprises the optical fibre 402, the reflective element 403 and additionally a beam splitter 503 associated to said reflective element 403. The beam splitter 503, functionally combined with the reflective element 403, allows to reflect the second light beam 524 coaxially with the axis M of the eye 100 thereby transmitting to the user eye only the normal polarization in order to reduce or cancel the corneal reflex.

## Claims

1. Optical accessory (201, 301) of a mobile device (30), comprising:
coupling means for the detachable association with said mobile device (30), wherein said coupling means allow the positioning of said optical accessory (201, 301) in correspondence with both a camera lens (32) and a light source (31) provided by said mobile device (30),
wherein said optical accessory further comprises:
a first optical element having a first reflective surface apt to reflect a source light beam (22) emitted by said light source (31) and to generate a first reflected beam substantially directed toward a visual field (V) of said camera lens (32);
a second optical element having a partially reflective second surface on which said first reflected beam is incident and apt to generate a second reflected beam directed toward an eye (100) to be examined inside the visual field (V) of said camera lens (32) in such a way to allow the view of the eyeground of said eye (100),
**characterised in that**:
said first and second optical elements have the shape of an optical guide element (202) which comprises at least one first face (203) identifying said first reflective surface, and at least one second face (204) identifying said partially reflective second surface, wherein said optical guide element (202) is a monoblock made of a transparent material, wherein at least said first face (203) has a polished surface, and at least said second face (204) has a satin-finished surface, which allows to generate said second light beam (224); or
wherein said first optical element is an optical guide element (302) which comprises at least one first face (303) identifying said first reflective surface apt to generate the first reflected beam (323), and a second substantially transparent face (304) opposite to said first face (303), wherein said optical guide element (302) is a monoblock made of a transparent material, and wherein said second optical element is a beam splitter (305) positioned in correspondence with said second face (304), so that when the first reflected beam (323) impinges on said beam splitter (305), said second generated reflected beam (324) is directed coaxially with the axis (M) of the eye (100) to be observed in the visual field (V) of said lens (32).

2. Optical accessory according to claim 1, wherein a first polarizing filter (225) is disposed at the exit of said first optical element (202, 302) and in combination with a second polarizing filter (226) positioned in the visual field of said lens (32).

3. Optical device comprising an optical accessory (10) according to claim 1 or 2, functionally associated to a mobile device (30) selected between a smartphone or a tablet, wherein said mobile device comprises a camera lens (32) and a light source (31).

## Patentansprüche

1. Optisches Zubehör (201, 301) einer mobilen Vorrichtung (30), umfassend: Kopplungsmittel zur lösbaren Verbindung mit der mobilen Vorrichtung (30), wobei die Kopplungsmittel die Positionierung des optischen Zubehörs ermöglichen (201, 301) im Bereich sowohl einer Kameralinse (32) als auch einer Lichtquelle (31), mit denen die mobile Vorrichtung (30) ausgerüstet ist, wobei das optische Zubehör ferner umfasst: ein erstes optisches Element mit einer ersten reflektierenden Oberfläche auf, die geeignet ist, einen von der Lichtquelle emittierten Quelllichtstrahl (22) zu reflektieren (31) und einen ersten reflektierten Strahl zu erzeugen, der im Wesentlichen auf ein Sichtfeld (V) der Kameralinse (32) gerichtet ist; ein zweites optisches Element mit einer teilweise reflektierenden zweiten Oberfläche, auf die der erste reflektierte Strahl trifft und die geeignet ist, einen zweiten reflektierten Strahl zu erzeugen, der zu einem zu untersuchenden Auge (100) gerichtet ist, das innerhalb des Sichtfeldes (V) der Kameralinse (32) derart enthalten ist, dass es die Sicht auf den Augenhintergrund (100) ermöglicht, **dadurch gekennzeichnet, dass** das erste und das zweite optische Element die Form eines optischen Führungselements (202) aufweisen, das mindestens eine erste Fläche (203), welche die erste reflektierende Oberfläche identifiziert, und mindestens eine zweite Fläche (204), welche die teilweise reflektierende zweite Oberfläche identifiziert, aufweist, wobei das optische Führungselement (202) ein Monoblock aus einem transparenten Material ist, wobei zumindest die erste Fläche (203) eine polierte Oberfläche aufweist, und zumindest die zweite Fläche (204) eine satinierte Oberfläche aufweist, die das Erzeugen des zweiten Lichtstrahles (224) ermöglicht; oder wobei das erste optische Element ein optisches Führungselement (302) ist, das mindestens eine erste Fläche (303), welche die zum Erzeugen des ersten reflektierten Strahles (323) geeignete erste reflektierende Oberfläche identifiziert, und eine zweite der ersten Fläche (303) gegenüberliegend angeordnete, im Wesentlichen transparente Fläche (304) aufweist, wobei das optische Führungselement (302) ein Monoblock aus einem transparenten Material ist, und wobei das zweite optische Element ein Strahlteiler (305) ist, der im Bereich der zweiten Fläche (304) derart positioniert ist, dass, wenn der erste reflektierte Strahl (323) auf den Strahlteiler (305) trifft, der zweite erzeugte reflektierte Strahl (324) koaxial zur Achse (M) des im Gesichtsfeld (V) der Linse (32) zu beobachtenden Auges (100) gerichtet ist.

2. Optisches Zubehör nach Anspruch 1, wobei am Ausgang des ersten optischen Elements (202, 302) ein erster Polarisationsfilter (225) und in Kombination damit ein zweiter im Sichtfeld der Linse (32) positionierter Polarisationsfilter (226) angeordnet ist.

3. Optische Vorrichtung umfassend ein optisches Zubehör (10) nach Anspruch 1 oder 2, die einer mobilen Vorrichtung (30) wie einem Smartphone oder einem Tablet funktionell zugeordnet ist, die eine Kameralinse (32) und eine Lichtquelle (31) umfasst.

## Revendications

1. Accessoire optique (201, 301) d'un dispositif mobile (30), comprenant:
des moyens de couplage pour la conjonction amovible avec ledit dispositif mobile (30), où lesdits moyens de couplage permettent le positionnement dudit accessoire optique (201, 301) en correspondance à la fois avec un objectif (32) de caméra et une source lumineuse (31) fournie par ledit dispositif mobile (30),
où ledit accessoire optique comprend en outre:
un premier élément optique ayant une première surface réfléchissante apte à réfléchir un faisceau lumineux (22) de source émis par ladite source lumineuse (31) et à générer un premier faisceau réfléchi dirigé sensiblement vers un champ visuel (V) dudit objectif (32) de caméra;
un deuxième élément optique ayant une deuxième surface partiellement réfléchissante sur laquelle est incident ledit premier faisceau réfléchi et qui est apte à générer un deuxième faisceau réfléchi dirigé vers un oeil (100) à examiner à l'intérieur du champ visuel (V) dudit objectif (32) de caméra de manière à permettre la vision du fond de l'oeil dudit oeil (100),
**caractérisé en ce que**: lesdits premier et deuxième éléments optiques ont la forme d'un élément de guidage optique (202) qui comprend au moins une première face (203) identifiant ladite première surface réfléchissante, et au moins une deuxième face (204) identifiant ladite deuxième surface partiellement réfléchissante, où ledit élément de guidage optique (202) est un monobloc en matériau transparent, où au moins ladite première face (203) a une surface polie, et au moins ladite deuxième face (204) a une surface satinée qui permet de produire ledit deuxième faisceau lumineux (224); ou
où ledit premier élément optique est un élément de guidage optique (302) qui comprend au moins une première face (303) identifiant ladite première surface réfléchissante apte à générer le premier faisceau réfléchi (323), et une deuxième face sensiblement transparente (304) opposée à ladite première face (303), où ledit élément de guidage optique (302) est un monobloc en matériau transparent, et où ledit deuxième élément optique est un séparateur (305) de faisceau placé en correspondance avec ladite deuxième face (304), de manière que lorsque le premier faisceau réfléchi (323) frappe sur ledit séparateur (305) de faisceau, ledit deuxième faisceau réfléchi généré (324) est dirigé coaxialement avec l'axe (M) de l'oeil (100) à observer dans le champ visuel (V) dudit objectif (32).

2. Accessoire optique selon la revendication 1, dans lequel un premier filtre polarisant (225) est disposé à la sortie dudit premier élément optique (202, 302) et en combinaison avec un second filtre polarisant (226) placé dans le champ visuel dudit objectif (32).

3. Dispositif optique comprenant un accessoire optique (10) selon la revendication 1 ou 2, fonctionnellement combiné avec un dispositif mobile (30) choisi entre un smartphone ou une tablette, dans lequel ledit dispositif mobile comprend un objectif (32) de caméra et une source lumineuse (31).
